# FASCICULE DE BREVET EUROPEEN

(11) **EP 0 694 525 B1**
(45) Date de publication et mention de la délivrance du brevet: **29.09.1999**
(21) Numéro de dépôt: 95201983.4
(22) Date de dépôt: 19.07.1995
(51) Int. Cl.: C07C 67/26, C07C 69/30, C07C 69/52, B01J 31/08

(54) **Procédé et installation pour la fabrication d'un ester de glycerol d'acide gras**
Verfahren und Einrichtung zum Herstellen von Fettsäureglycerolestern
Process and equipment for producing glycerol esters of fatty acids

(30) Priorité: 28.07.1994 FR 9409510
(43) Date de publication de la demande: 31.01.1996
(73) Titulaire: ORGANISATION NATIONALE INTERPROFESSIONNELLE DES OLEAGINEUX- ONIDOL, 75008 Paris (FR)
(72) Inventeur: Jamet, Jean-Paul, F-75012 Paris (FR); Staat, Frédéric, F-92700 Colombes (FR); Mouloungut, Zéphirin, F-31500 Toulouse (FR); Peyrou, Gilles, F-65500 Marsac (FR); Gachen, Christian, F-31500 Toulouse (FR); Rakotondrazafy, Vony, F-31500 Toulouse (FR); Gaset, Antoine, F-31000 Toulouse (FR)
(74) Mandataire: Cabinet BARRE LAFORGUE & associés

(56) Documents cités:
- EP-A- 0 545 477
- BE-A- 554 639
- BE-A- 657 517

## Description

L'invention concerne un procédé et une installation pour la fabrication d'un ester de glycérol d'acide gras. Le procédé visé est du type dans lequel on fait percoler un acide gras et du glycidol ou dérivé de glycidol à travers un lit de résine anionique forte macroporeuse jouant le rôle de catalyseur vis-à-vis de la réaction de condensation de l'acide gras avec le glycidol ou dérivé.

Ce procédé se caractérise par la nature de l'acide carboxylique de départ utilisé (acide gras), par la nature du coréactif (glycidol : composé comportant un oxygène sur le radical latéral fixé au groupe époxy) et par le mécanisme mis en oeuvre (ouverture du cycle époxy sur lequel l'acide gras vient se fixer). Ce type de réaction est fondamentalement différent et soulève des problèmes différents des réactions connues dans lesquelles l'acide est un acide éthylénique tel que acide acrylique ou bis-acrylique (et non un acide gras) en raison de la réactivité intrinsèque de ces acides éthyléniques qui conduisent facilement à la formation de carboxylate au contact des catalyseurs basiques (BE-A-657 517 ; US-A-3 404 174 ; US-A-4 970 333).

Il est décrit dans la demande de brevet FR 91.15117 un procédé de fabrication d'ester de glycérol du type précité dans lequel la fabrication s'effectue soit en milieu aqueux soit en milieu organique (éventuellement constitué par un excès d'acide gras). Dans ce document, il est indiqué qu'il est possible de travailler en continu en milieu aqueux ; par contre, une mise en oeuvre en continu en milieu organique n'est pas préconisée car elle soulève des difficultés résidant dans une tendance de la résine catalyseur à se déstructurer lors de la mise en contact de l'acide gras.

La présente invention se propose de fournir un procédé perfectionné permettant de fabriquer en continu ou semi-continu un ester de glycérol d'acide gras en milieu organique constitué par les réactifs eux-mêmes, c'est-à-dire en l'absence de tiers solvant (eau ou autre composé organique), et ce, sans dégradation de la résine catalyseur.

Un objectif de l'invention est ainsi de réduire le nombre et le volume des composés utilisés, à quantité d'ester produite égale, et d'augmenter la capacité de production d'une installation d'encombrement donné.

Un autre objectif est d'augmenter sensiblement le rendement en ester de la réaction et la concentration du produit obtenu, tout en conservant une sélectivité de la réaction proche de 100 % (le rendement étant défini comme le rapport du nombre de moles d'ester effectivement recueilli au nombre de moles théorique susceptible d'être obtenu par la réaction).

Un autre objectif est de favoriser l'obtention des monoesters au détriment des di et triesters afin de pouvoir profiter des propriétés spécifiques de ces monoesters qui permettent de les utiliser dans de nombreuses applications (émulsifiant, plastifiant, lubrifiant, agent anti-statique, agent transdermique, émollient, tensio-actif...).

A cet effet, le procédé de fabrication visé par l'invention, utilisant comme catalyseur une résine anionique forte macroporeuse, se caractérise en ce que :
(a) on dispose la résine à l'état humide en lit fixe et on fait percoler de l'eau déminéralisée à travers le lit,
(b) on procède a une phase de conditionnement consistant à faire percoler de l'acide gras à travers le lit de résine de façon à déplacer et éliminer l'eau contenue dans le lit, et à imprégner la résine au moyen dudit acide gras et engendrer une expansion du lit,
(c) on amène ensuite l'acide gras et le glycidol ou dérivé à percoler à travers le lit de résine ainsi imprégné de façon à effectuer la réaction en l'absence d'eau et de solvant,
(d) on recueille l'ester de glycérol formé.

On utilise de préférence une résine sous forme OH⁻, Cl⁻ ou HCO₃⁻. On constate de façon inattendue que le procédé sus-défini permet d'éviter toute dégradation de la résine qui passe du milieu humide initial au milieu acide gras sans déstructuration sensible. La phase de conditionnement au moyen de l'acide gras permet de chasser l'eau qui imbibe le lit et d'imprégner totalement la résine au moyen de cet acide ; au cours de cette phase, de l'acide gras est adsorbé sur la résine qui acquiert une grande stabilité thermique et chimique et une bonne activité. Il est important que seul l'acide gras soit amené à percoler pendant cette phase à l'exclusion du glycidol afin d'éviter un amorçage de la réaction de condensation. La condensation de l'acide gras avec le glycidol ou dérivé s'effectue ensuite au cours de la phase suivante : la réaction de condensation s'effectue en milieu réactif sans solvant étranger, de sorte que le volume des produits manipulés est réduit au strict minimum. La sélectivité en ester est de 100 % ; on constate en outre que le procédé de l'invention conduit à une sélectivité très élevée vis-à-vis du monoester (80 % à 100 %).

La mise en oeuvre du procédé peut être continue ou semi-continue. Dans le cas d une mise en oeuvre semi-continue, seul du glycidol ou dérivé est admis dans le lit lors de la phase réactive (c) et la percolation est de préférence réalisée jusqu'à l'admission dans le lit d'une quantité de glycidol ou dérivé correspondant à la stoechiométrie ; le milieu réactionnel est alors recueilli par vidange du liquide contenu dans le lit, et l'ester est ensuite séparé de ce milieu par des procédés classiques (chromatographie sur colonne en particulier). Dans le cas d'une mise en oeuvre continue :
. lors de la phase réactive (c), on amène en continu l'acide gras et le glycidol ou dérivé à percoler à travers le lit de résine de façon à effectuer la réaction de condensation en continu,

(d) on recueille en continu à la sortie du lit l'ester de glycérol formé.

Les avantages d'une telle mise en oeuvre continue résident essentiellement dans :
- une consommation totale du glycidol,
- un rendement plus élevé en ester de l'ordre de 80 % à 95 %,
- l'obtention d'une concentration en monoester qui peut être très élevée en sortie permettant une utilisation directe de ce composé dans de nombreuses applications,
- une mise en oeuvre plus simple du procédé et une capacité de production élevée rapportée à un volume donné de résine.

Selon un mode de mise en oeuvre préférentiel du procédé en continu, (c) on amène préalablement l'acide gras et le glycidol ou dérivé à se mélanger en continu, avantageusement dans des proportions sensiblement stoechiométriques, et on fait percoler le mélange à travers le lit de résine de façon que le temps de séjour de celui-ci soit compris entre 0,5 et 5 heures, le lit étant maintenu à une température comprise entre 40° C et 140° C.

La mise en oeuvre de la phase de conditionnement (b) peut elle-même être réalisée en continu en amenant l'acide gras à circuler en circuit fermé et en séparant l'eau entraînée par l'acide gras en aval du lit de résine. Dans ce cas, l'acide gras est avantageusement amené à circuler a travers le lit de façon que sa vitesse linéaire de passage soit comprise entre 5.10⁻⁴ m/s et 1,5.10⁻³ m/s, cette circulation étant poursuivie jusqu'à l'obtention d'un acide contenant moins de 1 % d'eau en poids à la sortie du lit.

Par ailleurs, l'opération (a) de percolation d'eau à travers la résine est de préférence réalisée avec un volume d'eau déminéralisée au moins égal à 5 fois le volume du lit de résine, la percolation étant poursuivie jusqu'à l'obtention d'un pH de l'eau compris entre 6,5 et 7 à la sortie du lit. Cette opération est avantageusement suivie d'un égouttage de l'eau en excès avant de procéder à la phase de conditionnement (b).

L'invention s'étend à une installation de fabrication continue d'un ester de glycérol, permettant de mettre en oeuvre le procédé précédemment défini. Cette installation comprend en combinaison :
. un réacteur tubulaire contenant un lit de résine anionique forte macroporeuse,
. des moyens de stockage d'acide gras,
. des moyens de stockage de glycidol ou dérivé,
. des moyens d'arrivée d'eau déminéralisée,
. des moyens de canalisation dotés de vannes pour la circulation de l'eau déminéralisée dans le réacteur tubulaire,
. des moyens de canalisation dotés de vannes et de moyens de pompage pour la circulation en circuit fermé de l'acide gras dans le réacteur tubulaire,
. des moyens de mélange d'acide gras et de glycidol ou dérivé, associés à des moyens de pompage et de dosage des produits entrants,
. des moyens de canalisation dotés de vannes et de moyens de pompage pour l'introduction en continu du mélange dans le réacteur et le recueil en continu des produits en sortie de celui-ci,
. des moyens de chauffage permettant de chauffer et thermostater le réacteur tubulaire, les moyens de stockage d'acide gras, les moyens de canalisation contenant de l'acide gras, les moyens de mélange, et les moyens de canalisation pour l'introduction du mélange et pour le recueil des produits.

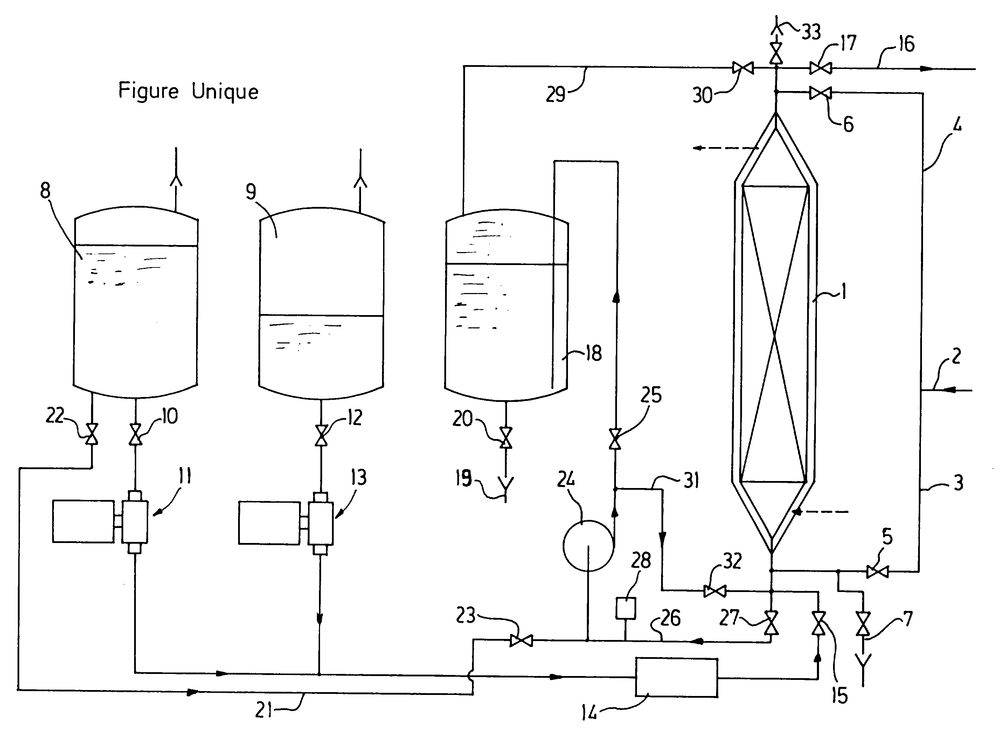

D'autres caractéristiques, buts et avantages ressortiront de la description qui suit, laquelle présente des exemples illustrant le procédé de l'invention pour la fabrication d'ester de glycérols d'acide gras à chaîne courte (C₇, C₉, C₁₁, C₁₂), à chaîne de longueur moyenne (C₁₄, C₁₆, C₁₈), et à chaîne longue (C₂₂) ; ces exemples ont été mis en oeuvre dans une installation à fonctionnement continu du type de celle représentée à la figure unique du dessin.

Cette installation comprend un réacteur tubulaire 1 à double paroi en vue de permettre de le chauffer et le thermostater. Ce réacteur de direction sensiblement verticale est rempli d'un lit de résine anionique forte macroporeuse sous forme OH⁻ ou Cl⁻ ou HCO₃⁻ selon les exemples.

Une arrivée d'eau déminéralisée schématisée en 2 est reliée à des conduits 3 et 4 dotés de vannes 5 et 6 qui permettent d'effectuer soit un remplissage ascendant du réacteur par admission de l'eau à sa partie basse, soit une percolation continue d'eau en sens inverse par admission de l'eau à sa partie haute et évacuation par sa partie basse dans un conduit de purge 7 doté d'une vanne.

L'installation comprend, en outre, une cuve 8 de stockage d'acide gras et une cuve 9 de stockage de glycidol (ou dérivé).

Ces cuves sont associées à des moyens de canalisations dotés de vannes et de moyens de pompage référencés respectivement en 10, 11 et 12, 13, qui permettent d'introduire des quantités dosées des produits dans un mélangeur 14. Ce dernier est relié à la base du réacteur 1 via une vanne 15 en vue de permettre une percolation ascendante du mélange.

Un conduit 16 de recueil des produits formés, doté d'une vanne 17, est relié à la partie haute du réacteur.

Par ailleurs, l'installation est complétée par un circuit permettant la mise en oeuvre de la phase de conditionnement de la résine après imprégnation d'eau.

Ce circuit comprend un circuit fermé agencé pour assurer une percolation d'acide gras dans le sens descendant : séparateur 18 de l'eau contenue dans le mélange acide gras/eau, conduit d'évacuation d'eau 19 et vanne 20 associés audit séparateur, conduit 21 d'acide gras relié à la cuve 8 via des vannes 22 et 23, moyens de pompage 24 de l'acide gras via une vanne 25 vers le séparateur 18, conduit 26 relié via une vanne 27, en partie basse du réacteur pour le recueil du mélange acide gras/eau, moyens 28 d'analyse de la teneur en eau du mélange, conduit 29 pour l'introduction de l'acide gras via une vanne 30 en partie haute du réacteur.

En outre, les moyens de pompage 24 sont rebouclés par un conduit 31 doté d'une vanne 32 sur la base du réacteur en vue de permettre une introduction d'acide gras dans le sens ascendant à l'intérieur du réacteur au début de la phase de conditionnement.

De plus, en partie haute du réacteur, un conduit 33 doté d'une vanne permet une mise à l'air libre du réacteur.

La cuve 8 de stockage d'acide gras et la cuve 18 du séparateur, et les conduits et moyens venant en contact avec l'acide gras ou l'ester formé sont associés, comme le réacteur, à des moyens classiques (non représentés aux fins de simplification) permettant de les maintenir à une température prédéterminée.

La mise en oeuvre d'une telle installation est la suivante :

### Phase (a) d'imprégnation d'eau déminéralisée

Un remplissage d'eau du réacteur dans le sens ascendant est d'abord réalisé en ouvrant les vannes 5 et 33, toutes les autres vannes étant fermées.

Lorsque le réacteur est plein d'eau déminéralisée, on assure une percolation d'eau dans le sens descendant en ouvrant les vannes 6 et 7, toutes les autres vannes étant fermées.

Lorsque le volume d'eau désiré a été amené à percoler le lit, on réalise un égouttage en ouvrant les vannes 33 et 7, toutes les autres vannes étant fermées.

### Phase (b) de conditionnement

On introduit d'abord de l'acide gras dans le réacteur dans le sens ascendant sur toute la hauteur du lit de résine en ouvrant les vannes 22, 23, 32 et 33 et en mettant la pompe 24 en marche, toutes les autres vannes étant fermées.

On remplit ensuite le séparateur 18 en ouvrant les vannes 22, 23, 25 et 30, toutes les autres vannes étant fermées (la pompe 20 restant en marche).

Enfin, on amène l'acide gras à percoler en continu dans le sens descendant dans le réacteur en ouvrant les vannes 30, 27 et 25, toutes les autres vannes étant fermées (la pompe 24 restant en marche).

### Phase réactionnelle (c)

Pour assurer la percolation du mélange acide gras/glycidol, et le recueil de l'ester formé, on ouvre les vannes 10, 12, 15 et 17 et l'on met en marche les moyens de pompage 11 et 13.

### EXEMPLE 1 : Fabrication en continu du monooléate de glycérol à partir de l'acide oléique à 97 %.

### Phase a :

La résine utilisée pour cet exemple est une résine "Amberlyst", marque déposée, réf. : A 26 OHE sous forme OH⁻, non tamisée fabriquée par "Rohm et Haas". Son humidité est de 61,1 % à la livraison (pourcentage pondéral d'eau rapporté au poids total de résine humide). 90 g de cette résine sont disposés dans le réacteur 1. A ce moment, on introduit l'eau déminéralisée en ouvrant la vanne 5 (toutes les autres vannes étant fermées sauf la vanne 33 qui sert de mise à l'air libre). L'alimentation se fait par un courant ascendant, ce qui permet un arrangement de la résine par fluidisation et l'élimination de l'air dans le lit de résine.

Lorsque le réacteur 1 est rempli à 80 % environ, on ferme les vannes 5 et 33 et on ouvre les vannes 6 et 7 de façon à faire percoler à travers le lit 750 cm³ d'eau déminéralisée par un courant descendant (5 fois le volume de résine environ) jusqu'à ce que le pH de l'eau soit égal à 6,5 à la sortie du lit.

Quand ce pH est atteint, on ferme la vanne 6 et on ouvre la vanne 33 jusqu'à égouttage total de la résine.

### Phase b :

Une fois le lit de résine égoutté, on ouvre les vannes 22, 23, 30 et 32, et par l'intermédiaire de la pompe 24 et des canalisations 29 et 31 on fait percoler l'acide oléique (à 97 %, provenance "Prolabo", marque déposée) à travers le lit et on remplit le séparateur 18.

Après cette opération, on ferme les vannes 22, 23 et 32 et on ouvre les vannes 25 et 27, le sens de circulation dans le réacteur 1 se trouvant alors inversé. La circulation s'effectue alors à travers le réacteur 1 (de bas en haut), les canalisations 29 et 31 et le séparateur 18. La vitesse de passage dans le réacteur 1 est de 0,67 10⁻³ m/s. On constate un entraînement d'eau ainsi qu'une décantation dans le séparateur 18. Cette opération s'effectue jusqu'à ce que la teneur en eau atteigne 1 % (on chauffe le réacteur 1 à 50° C lorsqu'on atteint 3 % d'eau dans l'acide oléique tout en refroidissant le séparateur 18).

### Phase c :

Lorsque la phase de conditionnement b est terminée, on augmente la température jusqu'à 70° C à l'intérieur du réacteur 1 et on ferme les vannes 25, 27 et 30, on arrête la pompe de circulation 24, on ouvre les vannes 10, 12 ; 19 et 17 et on met les pompes 11 et 13 en marche. Les deux réactifs sont amenés vers le réacteur 1 par l'intermédiaire des canalisations 34 et du mélangeur 14.

Le débit de l'acide oléique est de 33,27 g/h et celui du glycidol de 8,73 g/h, soit un débit total de 42 g/h.

### Phase d :

On recueille le monooléate de glycérol par l'intermédiaire de la canalisation 16.

### Résultats

- Débit total d'alimentation 42 g/h :
   . Acide oléique 32,3 g/h → 77 %
   . Glycidol 8,4 g/h → 20 %
   . Impuretés 1,3 g/h → 3 %
- Débit total de sortie 42 g/h :
   . Acide oléique 1,7 g/h → 4 %
   . Glycidol 0,0 g/h → 0 %
   . Monooléate de glycérol 36,1 g/h → 86 %
   . Di et trioléate de glycérol 0,0 g/h → 0 %
   . Autres 4,2 g/h → 10 %
Rendement en monooléate de glycérol : 89,6 %

### EXEMPLE 2 : Fabrication du monoglycéride de glycérol à partir des acides gras de tournesol oléique à 85 %

Les phases a, b, c et d sont identiques à celles de l'exemple 1. La composition de départ est :

| | |
|---|---|
| Acide oléique | 85 % |
| Acide stéarique | 5 % |
| Acide palmitique | 3 % |
| Acide linoléique | 2 % |
| Acide arachidique | 0,4 % |
| Acide béhénique | Traces |

### Résultats

- Débit total d'alimentation 42 g/h :
   . Acides gras 31,9 g/h → 76 %
   . Glycidol 8,4 g/h → 20 %
   . Impuretés 1,7 g/h → 4 %
- Débit total de sortie 42 g/h :
   . Acides gras 1,9 g/h → 4,5 %
   . Glycidol 0,0 g/h → 0 %
   . Monoglycérides de glycérol 35,3 g/h → 84 %
   . Di et triglycérides de glycérol 0,0 g/h → 0 %
   . Autres 4,8 g/h → 11,5 %
Rendement en monoglycérides de glycérol : 87,5 %

### EXEMPLE 3 : Fabrication du monoglycéride de glycérol à partir de l'acide oléique commercial à 65 %

Les phases a, b, c et d sont identiques à celles des exemples précédents.

### Résultats

- Débit total d'alimentation 41,5 g/h :
   . Acides gras 31,7 g/h → 76,5 %
   . Glycidol 8,3 g/h → 20,0 %
   . Impuretés 1,7 g/h → 3,5 %
- Débit total de sortie 41,5 g/h :
   . Acides gras 1,7 g/h → 4, %
   . Glycidol 0,0 g/h → 0 %
   . Monoglycérides de glycérol 35,7 g/h → 86 %
   . Di et triglycérides de glycérol 0,0 g/h → 0 %
   . Autres 4,2 g/h → 10 %
Rendement en monoglycérides de glycérol : 89,4 %

### EXEMPLE 4 : Fabrication du monooléate de glycérol à partir de l'acide oléique à 97 % en présence d'une résine anionique sous forme HCO⁻₃

Les phases a, b, c et d sont identiques à celles des exemples précédents, à l'exception de la résine anionique utilisée qui est une résine sous forme HCO⁻₃ obtenue à partir de la résine "Amberlyst A 26 OHE" qui a été conditionnée sous forme HCO⁻₃. La quantité de résine disposée dans le réacteur est la moitié de celle de l'exemple 1 (le taux d'humidité initial étant identique).

### Résultats

- Débit total d'alimentation 20,3 g/h :
   . Acide oléique 15,5 g/h → 76,4 %
   . Glycidol 4,1 g/h → 20,2 %
   . Impuretés 0,7 g/h → 3,4 %
- Débit total de sortie 20,3 g/h :
   . Acide oléique 1,0 g/h → 5 %
   . Glycidol 0,0 g/h → 0 %
   . Monooléate de glycérol 16,7 g/h → 82 %
   . Di et triglycérides 0,0 g/h → 0 %
   . Autres 2,6 g/h → 13 %
Rendement en monooléate de glycérol : 85,2 %

### EXEMPLE 5 : Fabrication du monooléate de glycérol à partir de l'acide oléique à 97 % en présence d'une résine sous forme chlorure.

Les phases a, b, c et d sont identiques à celles de l'exemple 4, à l'exception de la résine utilisée qui est sous la forme Cl⁻ ("Amberlyst A 26 Cl")

### Résultats

- Débit total d'alimentation 19,8 g/h :
   . Acide oléique 15,2 g/h → 76,8 %
   . Glycidol 4,0 g/h → 20,1 %
   . Impuretés 0,6 g/h → 3,0 %
- Débit total de sortie 19,8 g/h :
   . Acide oléique 0,8 g/h → 4,0 %
   . Glycidol 0,0 g/h → 0 %
   . Monooléate de glycérol 17,8 g/h → 90,0 %
   . Di et triglycérides 0,0 g/h → 0,0 %
   . Autres 1,2 g/h → 6,0 %
Rendement en monooléate de glycérol : 92,7 %

### EXEMPLE 6 : Fabrication du monoundécénoate de glycérol à partir de l'acide undécénoïque en présence d'une résine sous forme Cl⁻

Les phases a, b, c et d sont identiques à celles de l'exemple 5, la résine également. L'acide undécénoïque utilisé est pur.

### Résultats

- Débit total d'alimentation 13,5 g/h :
   . Acide undécénoïque 9,5 g/h → 70,48 %
   . Glycidol 3,8 g/h → 28,33 %
   . Autres 0,2 g/h → 1,19 %
- Débit total de sortie 13,5 g/h :
   . Acide undécénoïque 0,4 g/h → 3,0 %
   . Glycidol 0,0 g/h → 0,0 %
   . Monoundécénoate de glycérol 12,3 g/h → 91,0 %
   . Di et triglycérides 0,0 g/h → 0,0 %
   . Autres 0,8 g/h → 6,0 %
Rendement en monoundécénoate de glycérol : 93,2 %

### EXEMPLE 7 : Fabrication du monoheptanoate de glycérol à partir de l'acide heptanoïque en présence d'une résine sous forme Cl⁻

Les phases a, b, c et d sont identiques à celles des exemples 5 et 6, la résine également. L'acide heptanoïque utilisé est pur.

### Résultats

- Débit total d'alimentation 15 g/h :
   . Acide heptanoïque 9,41 g/h → 62,78 %
   . Glycidol 5,36 g/h → 35,73 %
   . Autres 0,23 g/h → 1,53 %
- Débit total de sortie 15 g/h :
   . Acide heptanoïque 0,6 g/h → 4,0 %
   . Glycidol 0,0 g/h → 0,0 %
   . Monoheptanoate de glycérol 13,8 g/h → 92,0 %
   . Di et triglycérides 0,0 g/h → 0,0 %
   . Autres 0,6 g/h → 4,0 %
Rendement en monoheptanoate de glycérol : 92 %

### EXEMPLE 8 : Fabrication du monoérucate de glycérol à partir de l'acide érucique à 90 %

Les phases a, b, c et d sont identiques à celles des exemples 1, 2 et 3.

### Résultats

- Débit total d'alimentation 45 g/h :
   . Acide érucique 34,9 g/h → 78 %
   . Glycidol 8,2 g/h → 18 %
   . Autres 1,9 g/h → 4 %
- Débit total de sortie 45 g/h :
   . Acide érucique 2,0 g/h → 4,5 %
   . Glycidol 0,0 g/h → 0,0 %
   . Monoérucate de glycérol 38,3 g/h → 85 %
   . Di et triérucate de glycérol 0,0 g/h → 0,0 %
   . Autres 4,7 g/h → 10,5 %
Rendement en monoérucate de glycérol : 88,9 %

### EXEMPLE 9 : Fabrication du monopélargonate de glycérol à partir de l'acide pélargonique à 95 %

Les phases a, b, c et d sont identiques à celles des exemples 1, 2, 3 et 8.

### Résultats

- Débit total d'alimentation 41 g/h :
   . Acide pélargonique 26,5 g/h → 64,7 %
   . Glycidol 12,5 g/h → 30,6 %
   . Autres 2,0 g/h → 4,7 %
- Débit total de sortie 41 g/h :
   . Acide pélargonique 1,2 g/h → 3 %
   . Glycidol 0,0 g/h → 0,0 %
   . Monopélargonate de glycérol 36,1 g/h → 88 %
   . Di et tripélargonate de glycérol 0,0 g/h → 0,0 %
   . Autres 3,7 g/h → 9 %
Rendement en monopélargonate de glycérol : 92,6 %

## Revendications

1. Procédé de fabrication d'un ester de glycérol, dans lequel on fait percoler un acide gras et du glycidol ou dérivé de glycidol à travers un lit de résine anionique forte macroporeuse jouant le rôle de catalyseur vis-à-vis de la réaction de condensation de l'acide gras avec le glycidol ou dérivé, ledit procédé étant caractérisé en ce que :
(a) on dispose la résine à l'état humide en lit fixe et on fait percoler de l'eau déminéralisée à travers le lit,
(b) on procède à une phase de conditionnement consistant à faire percoler de l'acide gras à travers le lit de résine de façon à déplacer et éliminer l'eau contenue dans le lit, et à imprégner la résine au moyen dudit acide gras et engendrer une expansion du lit,
(c) on amène ensuite l'acide gras et le glycidol ou dérivé à percoler à travers le lit de résine ainsi imprégné de façon à effectuer la réaction en l'absence d'eau et de solvant,
(d) on recueille l'ester de glycérol formé.

2. Procédé selon la revendication 1, caractérisé en ce que (a) on fait percoler à travers le lit un volume d'eau déminéralisée au moins égal à 5 fois le volume du lit de résine, la percolation étant poursuivie jusqu'à l'obtention d'un pH de l'eau compris entre 6,5 et 7 à la sortie du lit.

3. Procédé selon l'une des revendications 1 ou 2, dans lequel une opération d'égouttage de l'eau est réalisée avant la phase de conditionnement (b).

4. Procédé selon l'une des revendications 1, 2 ou 3, caractérisé en ce que (b) au cours de la phase de conditionnement, on fait circuler l'acide gras en circuit fermé, et on sépare l'eau entraînée par l'acide gras en aval du lit de résine.

5. Procédé selon la revendication 4, caractérisé en ce que, au cours de la phase de conditionnement (b), l'acide gras est amené à circuler à travers le lit de façon que sa vitesse linéaire de passage soit comprise entre 5.10⁻⁴ m/s et 1,5.10⁻³ m/s.

6. Procédé selon l'une des revendications 4 ou 5, dans lequel la phase de conditionnement par circulation d'acide gras (b) est poursuivie jusqu'à l'obtention d'un acide contenant moins de 1 % d'eau en poids à la sortie du lit.

7. Procédé selon l'une des revendications 1 à 6, caractérisé en ce que :
(c) on amène en continu l'acide gras et le glycidol ou dérivé à percoler à travers le lit de résine de façon à effectuer la réaction de condensation en continu,
(d) on recueille en continu à la sortie du lit l'ester de glycérol formé.

8. Procédé selon la revendication 7, caractérisé en ce que (c) on amène préalablement l'acide gras et le glycidol ou dérivé à se mélanger en continu et on fait percoler le mélange à travers le lit de résine de façon que le temps de séjour de celui-ci soit compris entre 0,5 et 5 heures, le lit étant maintenu a une température comprise entre 40° C et 140° C.

9. Procédé selon la revendication 8, dans lequel (c) on mélange en continu l'acide gras et le glycidol ou dérivé en proportion sensiblement stoechiométrique.

10. Procédé selon l'une des revendications 1 à 9, dans lequel l'on utilise une résine sous forme OH⁻, Cl⁻ ou HCO₃⁻.

11. Procédé selon l une des revendications 1 à 10, dans lequel on utilise un lit de résine s'étendant selon une direction sensiblement verticale, caractérisé en ce que :
a) on remplit le lit d'eau déminéralisée dans le sens ascendant et on fait ensuite percoler l'eau en sens inverse,
b) on introduit de l'acide gras dans le sens ascendant au moins sur la hauteur du lit et on inverse le sens de circulation de l'acide pour l'amener ensuite à percoler dans le sens descendant en vue d'entraîner leau.

12. Procédé selon l'une des revendications 1 à 11 pour la fabrication de monooléate de glycérol à partir de glycidol et d'acide oléique.

13. Installation de fabrication continue d'un ester de glycérol à partir d'acide gras et de glycidol ou dérivé, caractérisée en ce qu'elle comprend en combinaison :
. un réacteur tubulaire (1) contenant un lit de résine anionique forte macroporeuse,
. des moyens (8) de stockage d'acide gras,
. des moyens (9) de stockage de glycidol ou dérivé,
. des moyens (2) d'arrivée d'eau déminéralisée,
. des moyens de canalisation (3, 4) dotés de vannes (5, 6) pour la circulation de l'eau déminéralisée dans le réacteur tubulaire,
. des moyens de canalisation (21, 26, 27, 29, 31) dotés de vannes (22, 23, 25, 30, 32) et de moyens de pompage (24) pour la circulation de l'acide gras dans le réacteur tubulaire,
. des moyens (14) de mélange d'acide gras et de glycidol ou dérivé, associés à des moyens (11, 13) de pompage et de dosage des produits entrants,
. des moyens de canalisation dotés de vannes (15, 17) et de moyens de pompage (11, 13) pour l'introduction en continu du mélange dans le réacteur et le recueil en continu des produits en sortie de celui-ci,
. des moyens de chauffage permettant de chauffer et thermostater le réacteur tubulaire, les moyens de stockage d'acide gras, les moyens de canalisation de l'acide gras, les moyens de mélange, et les moyens de canalisation pour l'introduction du mélange et pour le recueil des produits.

14. Installation selon la revendication 13, caractérisée en ce que les moyens de canalisation pour la circulation de l'acide gras forment un circuit fermé (26, 29) comprenant un séparateur d'eau et d'acide gras (18).

15. Installation selon l'une des revendications 14 ou 15, dans laquelle le réacteur tubulaire (1) présente une direction sensiblement verticale, caractérisée en ce que :
. les moyens de canalisation pour la circulation de l'eau comprennent des conduits (4, 3) reliés à la partie haute et à la partie basse du réacteur en vue de permettre un remplissage ascendant de celui-ci et une percolation continue descendante,
. les moyens de canalisation pour la circulation d'acide gras comprennent des conduits (26, 29, 31) reliés à la partie haute et à la partie basse du réacteur en vue de permettre une introduction de l'acide gras par le bas et une percolation dans le sens descendant.

## Patentansprüche

1. Verfahren zur Herstellung eines Glycerolesters, bei dem eine Fettsäure und Glycidol oder ein Glycidolderivat über eine sehr poröse anionische Harzschicht geleitet wird, die als Katalysator für die Kondensationsreaktion der Fettsäure mit dem Glycidol bzw. dem Derivat fungiert, wobei das Verfahren durch die folgenden Schritte gekennzeichnet ist:
(a) Bereitstellen des Harzes im feuchten Zustand in einer festen Schicht und Perkolierenlassen von entmineralisiertem Wasser über die Schicht,
(b) Übergehen zu einer Aufbereitungsphase bestehend aus dem Perkolierenlassen von Fettsäure über die Harzschicht, um das in der Schicht enthaltene Wasser zu verdrängen und zu beseitigen und um den Harz mit der genannten Fettsäure zu imprägnieren und eine Expansion der Schicht zu erzeugen,
(c) anschließendes Zuführen der Fettsäure und des Glycidols bzw. des Derivats zum Perkolieren über die so imprägnierte Harzschicht, um die Reaktion in Abwesenheit von Wasser und von Lösungsmittel zu bewirken,
(d) Sammeln des gebildeten Glycerolesters.

2. Verfahren nach Anspruch 1, dadurch gekennzeichnet, daß (a) ein Volumen von entmineralisiertem Wasser von wenigstens dem Fünffachen des Volumens der Harzschicht über die Schicht perkoliert wird, wobei die Perkolation fortgesetzt wird, bis ein pH-Wert des Wassers im Bereich zwischen 6,5 und 7 am Ausgang der Schicht erreicht ist.

3. Verfahren nach einem der Ansprüche 1 und 2, bei dem vor der Aufbereitungsphase(b) ein Entwässerungsvorgang stattfindet.

4. Verfahren nach einem der Ansprüche 1, 2 und 3, dadurch gekennzeichnet, daß(b) im Laufe der Aufbereitungsphase die Fettsäure in einem geschlossenen Kreislauf zirkuliert und das von der Fettsäure mitgeführte Wasser unterhalb der Harzschicht abgeschieden wird.

5. Verfahren nach Anspruch 4, dadurch gekennzeichnet, daß im Laufe der Aufbereitungsphase(b) die Fettsäure so über die Schicht zirkuliert wird, daß ihre lineare Fließgeschwindigkeit zwischen 5 x 10⁻⁴ m/s und 1,5 x 10⁻³ m/s beträgt.

6. Verfahren nach einem der Ansprüche 4 und 5, bei dem die Aufbereitungsphase durch die Zirkulation von Fettsäure (b) fortgesetzt wird, bis eine Säure mit einem Wassergehalt von weniger als 1 Gew.-% am Ausgang der Schicht erhalten wird.

7. Verfahren nach einem der Ansprüche 1 bis 6, dadurch gekennzeichnet, daß:
(c) die Fettsäure und das Glycidol bzw. das Derivat im Durchlaufverfahren über die Harzschicht perkoliert werden, um die Kondensationsreaktion im Durchlaufverfahren zu bewirken,
(d) der gebildete Glycerolester im Durchlaufverfahren am Ausgang der Schicht gesammelt wird.

8. Verfahren nach Anspruch 7, dadurch gekennzeichnet, daß (c) die Fettsäure und das Glycidol bzw. das Derivat zuvor zum Vermischen im Durchlaufverfahren zugeführt werden und das Gemisch über die Harzschicht so perkolieren gelassen wird, daß dessen Aufenthaltszeit zwischen 0,5 und 5 Stunden beträgt, wobei die Schicht auf einer Temperatur zwischen 40°C und 140°C gehalten wird.

9. Verfahren nach Anspruch 8, bei dem(c) die Fettsäure und das Glycidol bzw. das Derivat in einem im wesentlichen stöchiometrischen Anteil im Durchlaufverfahren gemischt werden.

10. Verfahren nach einem der Ansprüche 1 bis 9, bei dem ein Harz in der Form OH⁻, Cl⁻ oder HCO₃⁻ verwendet wird.

11. Verfahren nach einem der Ansprüche 1 bis 10, bei dem eine Harzschicht verwendet wird, die in einer im wesentlichen senkrechten Richtung verläuft, dadurch gekennzeichnet, daß:
a) die Schicht im ansteigenden Sinn mit entmineralisiertem Wasser gefüllt und das Wasser dann im umgekehrten Sinn perkolieren gelassen wird,
b) Fettsäure im ansteigenden Sinn wenigstens bis zur Höhe der Schicht eingeleitet und die Zirkulationsrichtung der Säure umgekehrt wird, um sie dann im absteigenden Sinn zu perkolieren, um das Wasser mitzuführen.

12. Verfahren nach einem der Ansprüche 1 bis 11 für die Herstellung von Glycerolmonooleat aus Glycidol und Oleinsäure.

13. Vorrichtung zur kontinuierlichen Herstellung von Glycerolester aus Fettsäure und Glydidol oder einem Derivat davon, dadurch gekennzeichnet, daß sie in Kombination folgendes umfaßt:
. einen röhrenförmigen Reaktor (1), der eine sehr poröse anionische Harzschicht enthält,
. Mittel (8) zur Lagerung von Fettsäure,
. Mittel (9) zur Lagerung von Glycidol oder eines Derivats,
. Mittel (2) zum Einlassen von entmineralisiertem Wasser,
. Kanalisationsmittel (3, 4) mit Ventilen (5, 6) für die Zirkulation von entmineralisiertem Wasser in dem röhrenförmigen Reaktor,
. Kanalisationsmittel (21, 26, 27, 29, 31) mit Ventilen (22, 23, 25, 30, 32) und Pumpmittel (24) für die Zirkulation von Fettsäure in dem röhrenförmigen Reaktor,
. Mittel (14) zum Mischen von Fettsäure und Glycidol bzw. dem Derivat in Verbindung mit Mitteln (11, 13) zum Pumpen und Dosieren von Einleitungsprodukten,
. Kanalisationsmittel mit Ventilen (15, 17) und Pumpmitteln (11, 13) zum Einleiten des Gemisches im Durchlaufverfahren in den Reaktor und zum Sammeln der Produkte am Ausgang desselben im Durchlaufverfahren,
. Erhitzungsmittel zum Erhitzen und zur Temperaturregelung des röhrenförmigen Reaktors, der Fettsäure-Lagerungsmittel, der Fettsäure-Kanalisationsmittel, der Mischmittel und der Kanalisationsmittel zum Einleiten des Gemisches und zum Sammeln der Produkte.

14. Vorrichtung nach Anspruch 13, dadurch gekennzeichnet, daß die Kanalisationsmittel für die Zirkulation der Fettsäure einen geschlossenen Kreislauf (26, 29) bilden, der einen Wasser- und Fettsäureabscheider (18) beinhaltet.

15. Vorrichtung nach einem der Ansprüche 14 und 15, bei dem der röhrenförmige Reaktor (1) eine im wesentlichen senkrechte Richtung hat, dadurch gekennzeichnet, daß:
. die Kanalisationsmittel für die Zirkulation des Wassers Leitungen (4, 3) beinhalten, die im oberen Teil und im unteren Teil des Reaktors angeschlossen sind, um ein Auffüllen in aufsteigender Richtung desselben und ein ständiges absteigendes Perkolieren zuzulassen,
. die Kanalisationsmittel für die Fettsäurezirkulation Leitungen (26, 29, 31) beinhalten, die im oberen Teil und im unteren Teil des Reaktors angeschlossen sind, um ein Einleiten der Fettsäure von unten und eine Perkolation im absteigenden Sinn zuzulassen.

## Claims

1. Method for manufacturing a glycerol ester, wherein a fatty acid and glycidol or a glycidol derivative are made to percolate through a bed of macroporous strongly anionic resin acting as a catalyst in the condensation reaction between the fatty acid and glycidol or derivative, the said method being characterized in that:
(a) the resin is deposited in a moist state in a fixed bed and demineralized water is allowed to percolate through the bed,
(b) a conditioning phase is carried out consisting of allowing the fatty acid to percolate through the resin bed so as to displace and remove water contained in the bed and to impregnate the resin by means of the said fatty acid and to produce expansion of the bed,
(c) the fatty acid and glycidol or derivative are then allowed to percolate through the resin bed thus impregnated so as to carry out the reaction in the absence of water and solvent,
(d) the glycerol ester formed is recovered.

2. Method according to claim 1, characterized in that (a) a volume of demineralized water at least equal to 5 times the volume of the resin bed is allowed to percolate through the bed, percolation being continued until a pH of the water is reached of between 6.5 and 7 at the outlet from the bed.

3. Method according to either of claims 1 or 2, wherein a dewatering operation is carried out before the conditioning phase (b).

4. Method according to one of claims 1, 2 or 3, characterized in that (b) during the conditioning phase, the fatty acid is allowed to circulate in a closed circuit and water is separated entrained by the fatty acid upstream from the resin bed.

5. Method according to claim 4, characterized in that, during the conditioning phase (b), the fatty acid is allowed to circulate through the bed so that the linear rate of passage lies between 5×10⁻⁴ m/s and 1.5×10⁻³ m/s.

6. Method according to one of claims 4 or 5, wherein the conditioning phase by circulation of fatty acid (b) is continued until an acid is obtained containing less than 1 % water by weight at the outlet from the bed.

7. Method according to one of claims 1 to 6, characterized in that:
(c) the fatty acid and glycidol or derivative are allowed to percolate through the resin bed so as to carry out the condensation reaction continuously,
(d) the glycerol ester formed is recovered continuously at the outlet from the bed.

8. Method according to claim 7, characterized in that (c) the fatty acid and glycidol or derivative are first mixed continuously and the mixture is allowed to percolate continuously through the resin bed so that the dwell time therein lies between 0.5 and 5 hours, the bed being held at a temperature of between 40°C and 140°C.

9. Method according to claim 8, wherein (c) the fatty acid and glycidol or derivative are mixed continuously in a substantially stoichiometric proportion.

10. Method according to one of claims 1 to 9, wherein a resin is used in the OH⁻, Cl⁻ or HCO₃⁻ form.

11. Method according to one of claims 1 to 10, wherein a resin bed is used extending in a substantially vertical direction, characterized in that :
a) the bed is filled with demineralized water in an upward direction and water is then allowed to percolate in the reverse direction,
b) the fatty acid is introduced in an upward direction to at least the height of the bed and the direction of circulation of the acid is reversed so as then to allow it to percolate in a downward direction with a view to entraining water.

12. Method according to one of claims 1 to 11, for the manufacture of glycerol mono-oleate from glycidol and oleic acid.

13. Installation for the continuous manufacture of a glycerol ester from a fatty acid and glycidol or derivative, characterized in that it comprises, in combination:
. a tubular reactor (1) containing a bed of macroporous strongly anionic resin,
. means (8) for storing the fatty acid,
. means (9) for storing glycidol or derivative,
. means (2) for supplying demineralized water,
. pipelines (3, 4) provided with valves (5, 6) for circulating demineralized water through the tubular reactor,
. pipelines (21, 26, 27, 29, 31) provided with valves (22, 23, 25, 30, 32) and means of pumping (24) for circulating the fatty acid through the tubular reactor,
. means (14) for mixing the fatty acid and glycidol or derivative, associated with means (11, 13) for pumping and metering the entering products,
. pipe lines provided with valves (15,17) and means of pumping (11,13) for introducing the mixture continuously into the reactor and for collecting the products continuously at the outlet therefrom,
. means of heating enabling the tubular reactor, the means for storing the fatty acid, the pipeline for the fatty acid, the means of mixing and the pipelines for introducing the mixture and for recovering the product, to be heated and temperature-controlled.

14. Installation according to claim 13, characterized in that the pipelines for circulating the fatty acid form a closed circuit (26,29) including a separator for water and fatty acid (18).

15. Installation according to one of claims 14 or 15, wherein the tubular reactor (1) has a substantially vertical direction, characterized in that :
. the pipelines for circulating water include pipes (4,3) connected to the upper part and the lower part of the reactor with a view to enabling the latter to be filled upwards and for percolation to take place continuously downwards,
. the pipelines for circulating fatty acid include pipes (26,29,31) connected to the upper part and the lower part of the reactor with a view to allowing fatty acid to be introduced through the bottom and percolation to take place in a downwards direction.
